# EUROPÄISCHE PATENTSCHRIFT

(11) **EP 0 730 860 B1**
(45) Veröffentlichungstag und Bekanntmachung des Hinweises auf die Patenterteilung: **02.01.2002**
(21) Anmeldenummer: 96103363.6
(22) Anmeldetag: 05.03.1996
(51) Int. Cl.: A61K 9/107, A61K 9/127

(54) **Mischmicellen**
Mixed micelles
Micelles mixtes

(30) Priorität: 07.03.1995 CH 65495
(43) Veröffentlichungstag der Anmeldung: 11.09.1996
(73) Patentinhaber: F. HOFFMANN-LA ROCHE AG, 4070 Basel (CH)
(72) Erfinder: Pittrof, Folker, 79618 Rheinfelden (DE); Steffen, Hans, 4410 Liestal (DE)
(74) Vertreter: Vergani, Diego, Dr.

(56) Entgegenhaltungen:
- EP-A- 0 011 745
- EP-A- 0 154 977
- EP-A- 0 280 887
- EP-A- 0 388 817
- EP-A- 0 439 042
- EP-A- 0 471 309
- WO-A-90/08534
- WO-A-91/07170
- DE-A- 2 730 570
- CHEMICAL ABSTRACTS, Band 115, Nr. 7, 19. August 1991, Columbus, Ohio, USA FOERSTER G. et al. "Comments on: Phase diagrams and structures of lecithin/water systems with branched lecithin components" Seite 340, Spalte 1, Nr. 67 073n; & Colloid Polym. Sci. 1991, 269(4), 412-14 (Eng)

## Beschreibung

Die vorliegende Erfindung betrifft neue Mischmicellen und Kompositionen, die solche Mischmicellen enthalten.

Mischmicellen aus Gallensäuresalzen und natürlichen Phosphatiden wie Ei- oder Soyalecithin und Kompositionen, die solche Mischmicellen enthalten, sind z.B. aus der Patentpublikation DE-A-27 30 570 bekannt. Derartige Micellsysteme können zur wässrigen Solubilisierung von in Wasser schlecht oder nicht löslichen Wirkstoffen, z.B. pharmazeutischen Wirkstoffen in parenteral verabreichbaren Formulierungen, zur Verbesserung der parenteralen Verträglichkeit bei wasserlöslichen Wirkstoffen (vgl. EP-A-0280 887) oder zur Verbesserung der Hautpenetration von pharmazeutischen Wirkstoffe oder Kosmetika verwendet werden (vgl. EP-A-0 439 042). Die Europäische Patentanmeldung EP 154 977 beschreibt Glycerinätherphosphatide, die für die Vorbereitung der oben erwähnten Mischmicellsysteme verwendet werden können. Die Anwesenheit ungesättigter Fettsäuren in natürlichen Phosphatiden kann zu Peroxidbildung führen, die auch bei Zusatz von Antioxidantien und Chelatisierungsmitteln die Stabilität oxidationsempfindlicher Wirkstoffe, die in diesen Formulierungen enthalten sind, beeinträchtigen können. Den natürlichen Phosphatiden entsprechende Phosphatide mit gesättigten Fettsäureresten würden den Nachteil der Peroxidbildung vermeiden. Mischmicellen existieren jedoch nur oberhalb einer kritischen Temperatur, der

Phasenumwandlungstemperatur, die bei Phosphatiden mit gesättigten langkettigen Fettsäureresten wesentlich höher liegt als z.B. bei Soya- oder Eilecithin (z.B. bei etwa 50°C für voll hydriertes Soyalecithin im Gegensatz zu etwa -18°C für das nicht hydrierte Produkt). Dieser Umstand steht der Verwendung z.B. von perhydriertem Soyalecithin für die Herstellung von Mischmicellen für Lagerungstemperaturen von Raumtemperatur oder darunter entgegen.

Lecithine mit kurzkettigen Carbonsäuren (< C8) besitzen eine niedrige Phasenübergangstemperatur, so dass prinzipiell Mischmicellen bei Raumtemperatur herstellbar sind. Diese Lecithine sind jedoch zu toxisch bzw. hämolytisch, um parenteral angewandt werden zu können, jedoch wurden Mischmicellen aus Dihexanoyllecithin und Ei- oder Sojalecithin für solche Anwendungszwecke vorgeschlagen (vgl. EP-A-0011745).

Dilaurylphosphatidylcholin (DLPC) zeigt eine Phasenübergangstemperatur von ca. 0°C und besitzt keine hämolytische Eigenaktivität. Es fehlt jedoch der für Ei- und Sojalecithin typische und für die Verwendung in Gallensalzmischmicellen wichtige antihämolytische Effekt, so dass DLPC-Gallensalz-Mischmicellen ebenfalls nicht parenteral eingesetzt werden.

Es wurde nun überraschend gefunden, dass Mischmicellen im Bereich von Normal(Raum)temperatur oder darunter mit Phosphatiden, die gesättigte Fettsäurereste enthalten, gebildet werden, wenn die Fettsäurereste verzweigt sind.

Die Erfindung betrifft infolgedessen Mischmicellen, die aus
a) einem Phosphatid mit gesättigten, verzweigten Fettsäureresten und
b) einem Gallensäuresalz oder Dihexanoyllecithin gebildet sind.

Die Erfindung betrifft weiterhin die Verwendung derartiger Mischmicellen zur wässrigen Solubilisierung von in Wasser schlecht oder nicht löslichen Wirkstoffen, z.B. pharmazeutischen Wirkstoffen in parenteral verabreichbaren Formulierungen, zur Verbesserung der parenteralen Verträglichkeit bei wasserlöslichen Wirkstoffen oder zur Verbesserung der Hautpenetration von pharmazeutischen Wirkstoffe oder Kosmetika, weiterhin in Diagnostica zur Stabilisierung von Proteinen, wie z.B. membrangebundenen Rezeptoren.

Vorzugsweise enthalten die Phosphatide verzweigte gesättigte Fettsäurereste mit 14-20 C-Atomen in gerader Kette und haben eine Phasenumwandlungstemperatur unterhalb 20°C, vorzugsweise unterhalb 10°C. Die Verzweigung kann einfach oder mehrfach sein, bevorzugt sind einfach verzweigte gesättigte Fettsäurereste, z.B. durch eine Methyl- oder Aethylgruppe einfach substituierte Fettsäurereste. Bevorzugt sind die Fettsäurereste im Mittelteil der Kette, z.B. in Stellung 8, 10 oder 12 substituiert bzw. verzweigt. Weiterhin sind Phosphatide bevorzugt, die Fettsäurereste mit einer ungeraden Anzahl C-Atomen in gerader Kette enthalten. Beispiele von erfindungsgemässen gesättigten, verzweigten Fettsäureresten sind der 10-Methylnonadecanoyl-, der 10-Methylstearoyl-, der 8-Methylheptadecanoyl, der 8-Methylpalmitoyl- und der 10-Ethylstearoylrest. Beispiele erfindungsgemäss verwendbarer Phosphatide sind somit
1,2-Di(10-methylnonadecanoyl)-sn-glycero-3-phosphocholin,
1,2-Di(10-methylstearoyl)-sn-glycero-3-phosphocholin,
1,2-Di(8-methylheptadecanoyl)-sn-glycero-3-phosphocholin,
1,2-Di(8-methylpalmitoyl)-sn-glycero-3-phosphocholin, und
1,2-Di(10-ethylstearoyl)-sn-glycero-3-phosphocholin,
sowie Enantiomere davon. Weitere Beispiele erfindungsgemäss verwendeter Phosphatide sind Phosphatidylethanolamine mit gesättigten, verzweigten Fettsäureresten, z.B.
1,2-Di(10-methylnonadecanoyl)-sn-glycero-3-phosphoethanolamin,
1,2-Di(10-methylstearoyl)-sn-glycero-3-phosphoethanolamin,
1,2-Di(8-methylheptadecanoyl)-sn-glycero-3-phosphoethanolamin,
1,2-Di(8-methylpalmitoyl)-sn-glycero-3-phosphoethanolamin, und
1,2-Di(10-ethylstearoyl)-sn-glycero-3-phosphoethanolamin.

Von besonderem Interesse ist das 1,2-Di(10-methylnonadecanoyl)-sn-glycero-3-phosphocholin, mit dem Mischmicellen auch unter Kühlschrankbedingungen gebildet werden.

Die erfindungsgemässen Mischmicellen können in wässriger Lösung aus den Komponenten, d.h., aus einem Gallensäuresalz oder Dihexanoyllecithin, und einem Phosphatid mit gesättigten, verzweigten Fettsäureresten in an sich bekannter Weise, z.B. wie in den Patentpublikationen DE-A-27 30 570 und WO 90/08534 beschrieben hergestellt werden, beispielsweise durch Vermischen der einzelnen Bestandteile oder dadurch, dass man die Micellbildner und einen gegebenenfalls beizufügenden Wirkstoff in einem organischen Lösungsmittel, z.B. Methylenchlorid, Aethanol und/oder Methanol löst, das Lösungsmittel, zweckmässig in einem Rotationsverdampfer, entfernt und den Rückstand in Wasser oder einer wässrigen Lösung von pharmazeutischen Hilfsstoffen, wie Pufferstoffen oder Isotonisierungsmitteln löst; oder die freien Gallensäuren, gegebenenfalls unter Zusatz pharmazeutischer Hilfsstoffe und/oder wasserlöslicher Wirkstoffe, in Wasser suspendiert, das Phosphatid und gegebenenfalls pharmazeutische Wirkstoffe oder Kosmetika in der Suspension dispergiert und das Produkt mit einer Base, z.B. einem Alkalihydroxid, neutralisiert.

Bevorzugt sind Mischmicellen, die ein Gallensäuresalz enthalten.

Beispiele von Gallensäuresalzen, die für die erfindungsgemässen Mischmicellen in Betracht kommen sind Alkalisalze, wie Natriumsalze von Cholsäure, Glycocholsäure, Taurocholsäure, Deoxycholsäure, Glyco- oder Taurodeoxycholsäure, Chenodeoxycholsäure und Glyco- oder Taurochenoxydeoxycholsäure. Bevorzugt ist Natrium-Glycocholat. Das molare Verhältnis von Phosphatid : Gallensäuresalz beträgt zweckmässig etwa 0,1:1 bis 2:1, vorzugsweise 0,8:1 bis 1,5:1. Die Gallensalzkonzentration soll jedoch immer oberhalb der gallensalzspezifischen kritischen Micellbildungskonzentration (CMC) liegen.

Besonders bevorzugt sind Mischmicellen aus 1,2-Di (10-methylnonadecanoyl)-sn-glycero-3-phosphocholin und Natrium-Glycocholat.

Die erfindungsgemässen Mischmicellen können gewünschtenfalls pharmazeutische Wirkstoffe für parenterale, topische oder orale Verabreichung enthalten, insbesondere in Wasser nicht oder schwer lösliche Wirkstoffe, oder Kosmetika, sowie Hilfsstoffe, wie Puffer, z.B. Phosphat-, Citrat- oder Tris-Puffer; oder Isotonisierungsmittel z.B. Natriumchlorid, Mannitol, Sorbitol oder Glucose; odere Konservierungsmittel, z.B. Methyl- oder Propyl-p-hydroxybenzoat, Benzylalkohol oder Phenol. Beispiele von pharmazeutischen Wirkstoffen, die in den erfindungsgemässen Mischmicellen enthalten sein können, sind Retinoide wie all trans, 13-cis und 9-cis Retinsäure und deren Derivate inkl. aromatische Verbindungen wie Etretinat, Vitamin K, Vitamin D und deren Derivate; peroxidhaltige Wirkstoffe wie Artemisinin, Arteflene und Benzoylperoxid; wasserlösliche niedermolekulare und hochmolekulare Wirkstoffe wie z.B. Pyrimidinanaloge (5-Fluoruracil); Heparin; und Proteine. Beispiele solcher Proteine sind Interferone (IFN), wie IFN-α, IFN-β, IFN-γ Hybridinterferone; Interleukine (IL), wie IL-1 (ETAF, LAF), IL-2 (TCGF), IL-3 (Multi-CSF, MCGF), IL-4 (BSF-1, BCGF-1), IL-5 (TRF, BCGF-II), IL-7 (Lymphopoietin 1); Lymphotoxin (TNF-β); Macrophage Inhibitory Factor (MIF); Thymopoietin (TPO); Transforming Growth Factor-α (TGF-α); Transforming Growth Factor-β (TGF-β); Tumor Nekrose Faktor (TNF-α, Cachectin, DIF); Uromodulin (Tamm-Horsfall-Protein); Neuroleukin; CD4; Proteine mit Wirkung auf Immunfunktionen, wie Granulocyte Colony Stimulating Factor (G-CSF), Granulocyte-Macrophage Colony Stimulating Factor (GM-CSF, CSF-2), Macrophage Colony Stimulating Factor (CSF-1, M-CSF); Antikörper oder Antikörper-Arzneistoff-Konjugate, Hybridproteine wie IL-2-Diphterietoxin und Proteine zur Herstellung von Vakzinen gegen AIDS, Malaria, Hepatitis, Herpes, Influenza, Poliomyelitis und andere Infektionskrankheiten.

Beispiele von Kosmetika die in den erfindungsgemässen Mischmicellen enthalten sein können, sind Vitamin E, Panthenol und Feuchthaltemittel wie Milch- und Pyrrolidoncarbonsäure und deren Salze.

Die erfindungsgemässen Mischmicellen können in wässriger oder organisch-wässriger Lösung oder als Lyophilisate solcher Lösungen vorliegen. In wässrigen Lösungen können die Mischmicellen in einer Konzentration von bis zu etwa 200 mg Phosphatid/ml enthalten sein. Die Mischmicell-Lösungen können pharmazeutische Wirkstoffe oder Kosmetika vorzugsweise in Mengen von bis zu etwa 20 mg/ml enthalten.

Phosphatide mit verzweigten Fettsäureresten sind generell bekannt. Sie kommen z.B. in der Muttermilch und in Hautlipiden vor. Sie können z.B. durch partielle Veresterung von Glycerin mit den entsprechenden gesättigten, verzweigten Fettsäuren zum Di-ester, Posphorylierung des Diesters und Umsetzung mit einem geeigneten Cholinsalz, wie Cholin-tosylat, wie nachstehend beschrieben oder in Analogie dazu hergestellt werden.

Die Erfindung wird durch die nachstehenden Beispiele weiter erläutert.

### Beispiel 1

Herstellung von 1,2-Di(10-methylnonadecanoyl)-sn-glycero-3-phosphocholin:
a) Zu einer Lösung von 90,2 g 10-Methyl-nonadecansäure (70%) und 21,0 g (R)-Benzyloxy-1,2-propandiol in 2 L Methylenchlorid wurden unter Rühren 59,8 g Dicyclohexylcarbodiimid und 35,4 g 4-Dimethylamino-pyridin gegeben. Dann wurde 24 Stunden bei Raumtemperatur gerührt. Der ausgefallene Harnstoff wurde abfiltriert, dreimal mit je 200 ml n-Hexan nachgewaschen, und die vereinigten Filtrate bei 35°C im Wasserstrahlvakuum eingeengt. Das Rohprodukt wurde mit 500 ml n-Hexan versetzt und unter Rühren auf 40°C erwärmt und danach ebenfalls unter Rühren auf 0-5°C abgekühlt. Der ausgefallene Harnstoff wurde nochmals mit 250 ml n-Hexan nachgewaschen und das Filtrat bei 35°C im Wasserstrahlvakuum eingedampft. Der Rückstand wurde in 210 ml n-Hexan gelöst und an 2,5 kg Kieselgel in 10,5 l n-Hexan mit ca. 23 l n-Hexan/Essigester (95:5) chromatographiert. Die Produkt enthaltenden Fraktionen wurden vereinigt, eingeengt, in 100 ml Toluol gelöst und über 4 kg Kieselgel zuerst mit ca. 35 l Toluol und dann mit ca. 15 l n-Hexan/Essigester (95:5) gereinigt. Die chromatographisch einheitlichen Fraktionen wurden vereinigt und am Rotationsverdampfer im Wasserstrahlvakuum bei 40°C eingeengt.
b) 56,5 g des in a) erhaltenen Diesters wurden in 2 l Essigester gelöst, mit 5,6 g Palladium/Kohle 5% versetzt und über Nacht bei Raumtemperatur hydriert. Das Reaktionsgemisch wurde über 300 g Natriumsulfat filtriert und mit 1,5 l Essigester nachgewaschen. Die vereinigten Essigesterphasen wurden im Wasserstrahlvakuum bei 40°C eingeengt. Man erhielt ein leicht gelbliches Oel.
c) 20 g des in b) erhaltenen Oels wurden unter Argon und unter Rühren in 280 ml Chloroform bei Raumtemperatur gelöst. Danach wurden bei dieser Temperatur unter Rühren 4 ml (43,6 mMol) Phosphoroxychlorid und 2 Minuten später 5,2 ml (43,6 mMol) Chinolin zugesetzt. Das Reaktionsgemisch wurde 2 Stunden bei 30°C gerührt, danach auf Raumtemperatur abgekühlt und dann zuerst mit 17,8 g Chinolintosylat und dann mit 16,8 ml Pyridin versetzt. Das Reaktionsgemisch wurde über Nacht (16-18 Stunden) bei Raumtemperatur gerührt. Zu dem Reaktionsgemisch wurden dann innerhalb von 5 Minuten unter weiterem Rühren 44 ml entionisiertes Wasser zugetropft und anschliessend mit 11,2 g Natriumhydrogencarbonat versetzt und 30 Minuten nachgerührt. Das Reaktionsgemisch wurde mit 250 ml Chloroform und 100 ml Methanol versetzt und kräftig geschüttelt, die organische Phase abgetrennt und nacheinander mit 260 ml entionisiertem Wasser/Methanol (8:5), 290 ml 1N Salzsäure/Methanol (16:13), 320 ml entionisiertem Wasser/Methanol (1:1) und nochmals 320 ml entionisiertem Wasser/Methanol (1:1) gewaschen. Die wässrigen Phasen wurden nacheinander mit 100 ml Chloroform rückextrahiert. Die vereinigten Chloroform-Phasen wurden über Natriumsulfat getrocknet und bei 40°C im Wasserstrahlvakuum eingeengt. Das zurückbleibende Oel wurde in 300 ml Methanol aufgenommen, 10 Minuten bei 30-35°C gerührt und dann auf 0°C abgekühlt und 1 Stunde bei dieser Temperatur gerührt. Nach 20 Minuten Stehen im Eisbad wurde das abgeschiedene Oel abgetrennt. Das klare Filtrat wurde auf -5°C gekühlt, 10 Minuten bei dieser Temperatur belassen und das ausgeschiedene Oel wiederum abgetrennt. Der Ueberstand wurde bei 40°C im Wasserstrahlvakuum eingeengt. Der Rückstand wurde in 100 ml Diethylether gelöst und unter Rühren mit 150 ml Aceton versetzt, der Ether bei 35°C entfernt und das Oel nochmals in 130 ml Diethylether gelöst und mit 120 ml Aceton versetzt. Nach Entfernen des Ethers bei 35°C wurde der Rückstand mit 50 ml Aceton versetzt, auf 18°C gekühlt und vom ausgefallenen Oel abdekantiert. Das so erhaltene ölige 1,2-Di(10-methylnonadecanoyl)-sn-glycero-3-phosphocholin wurde bei Raumtemperatur im Vakuum bei 1 mbar getrocknet.

### Beispiel 2

Herstellung einer Mischmicell-Lösung:

Aus einer Stammlösung von 20 ml 0,1 m 1,2-Di(10-methylnonadecanoyl)-sn-glycero-3-phosphocholin in Methylenchlorid und 20 ml 0,1 m Na-Glycocholat in Methanol wurde durch Eindampfen der Lösungsmittel und Aufnehmen des als Film zurückbleibenden Rückstandes in 2 ml demineralisiertem Wasser eine Mischmicell-Lösung hergestellt.

### Beispiel 3

Es wurden konventionelle und erfindungsgemässe Mischmicell-Lösungen der nachstehenden Zusammensetzung hergestellt:

| | /01 | /02 | /03 | /04 | /05 | |
|---|---|---|---|---|---|---|
| 9-cis-Retinsäure | 0,5 | 0,5 | 0,5 | 0,5 | 0,5 | mg |
| Ethanol 94% (w/w) | 100,0 | 100,0 | 100,0 | 100,0 | 100,0 | mg |
| Glycocholsäure | 88,5 | 88,5 | 88,5 | 88,5 | 88,5 | mg |
| Iso-Lecithin ∗ | 163 | 163 | | | | mg |
| Soya-Lecithin | | | 152 | 152 | 152 | mg |
| NaOH ad | pH 6,0 | pH 6,0 | pH 6,0 | pH 6,0 | pH 6,0 | |
| Benzylalkohol | 10,0 | 10,0 | 10,0 | 10,0 | 10,0 | mg |
| dl-α-Tocopherol | -- | 10,0 | -- | 10,0 | 10,0 | µg |
| Ascorbinsäure | -- | 50,0 | -- | -- | 50,0 | µg |
| Wasser ad | 1,0 | 1,0 | 1,0 | 1,0 | 1,0 | ml |

| | | | | | | |
|---|---|---|---|---|---|---|
| ∗ 1,2-Di-(10-methylnonadecanoyl)-sn-glycero-3-phosphocholin | | | | | | |

Zur Herstellung der Lösungen wurden Ethanol und Wasser entgast und alle Manipulationen unter Rotlicht und Stickstoff ausgeführt. Die wasserlöslichen Excipientien wurden in Wasser gelöst und das pH auf 6.0 eingestellt. 9-cis-Retinsäure und Tocopherol wurden in Ethanol gelöst. Die Lösung der wasserlöslichen Stoffe wurde zu der ethanolischen Lecithin-Lösung gegeben und bis zur Bildung einer klaren Lösung gerührt (18 h). Danach wurde die ethanolische Lösung von 9-cis-Retinsäure zugegeben und das Volumen eingestellt. Die so erhaltene Mischmicell-Lösung wurde in Flaschen mit Teflon-Stopfen abgefüllt und 20 min bei 120 °C sterilisiert. Die chemische Stabilität der 9-cis Retinsäure in diesen Lösungen nach 3 Monaten Lagerung bei verschiedenen Temperaturen ist aus Tabelle 1 ersichtlich. Die Ergebnisse zeigen, dass in den erfindungsgemässen Lösungen im Gegensatz zu den konventionellen Lösungen kein oxidativer Abbau der 9-cis-Retinsäure eingetreten war. Der Verlust an 9-cis-Retinsäure in den erfindungsgemässen Lösungen nach Sterilisation ist nicht auf oxidativen Abbau sondern auf eine verstärkte Isomerisierung zurückzuführen.

Es konnte weiterhin gezeigt werden, dass die erfindungsgemässen Mischmicellen wie z.B. die in Beispiel 3 beschriebenen Formulierung /02 in ihrer hämolytischen resp. antihämolytischen Aktivität mit einr konventionellen Mischmicelle wie /05 in Beispiel 3 vergleichbar ist.

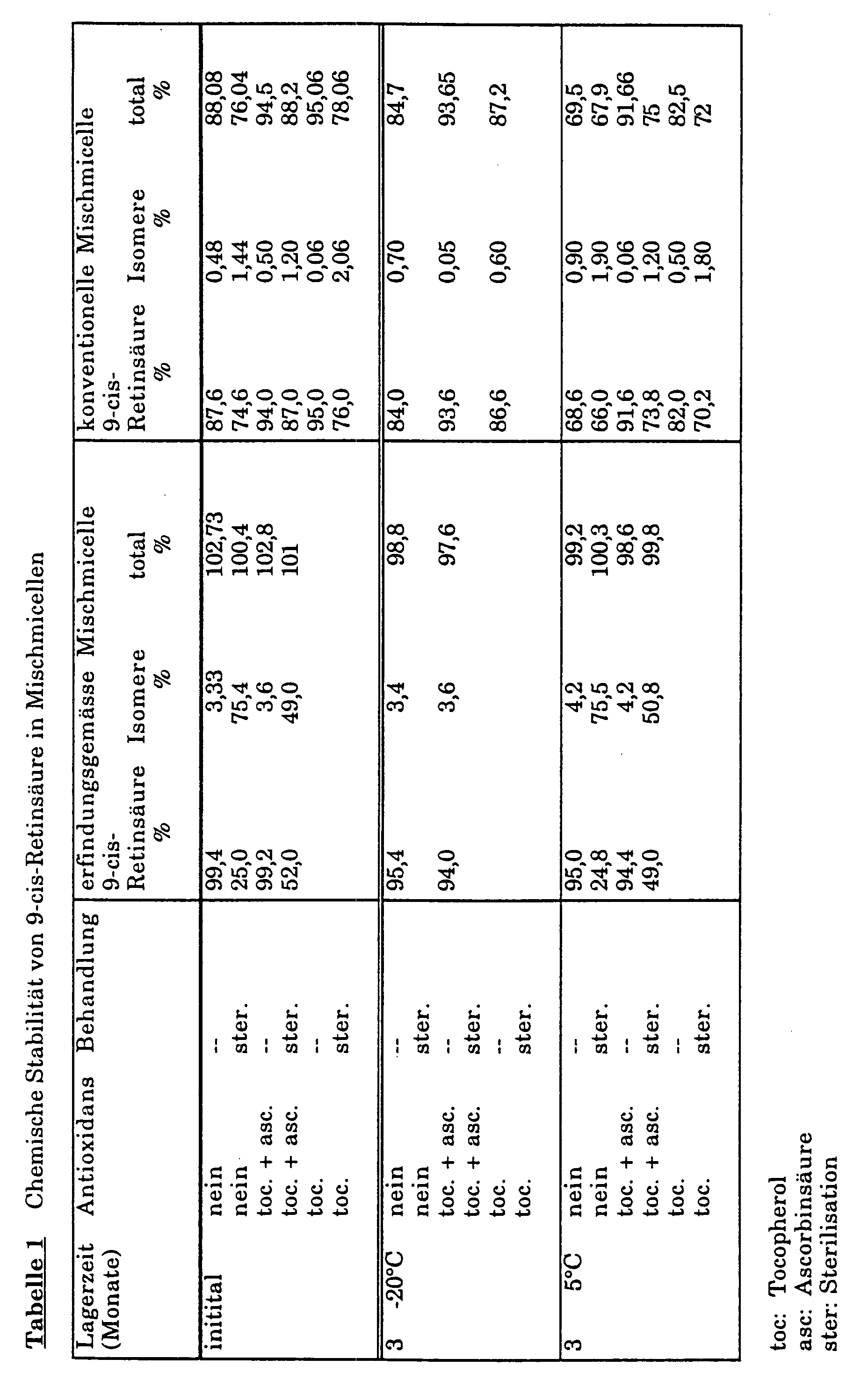

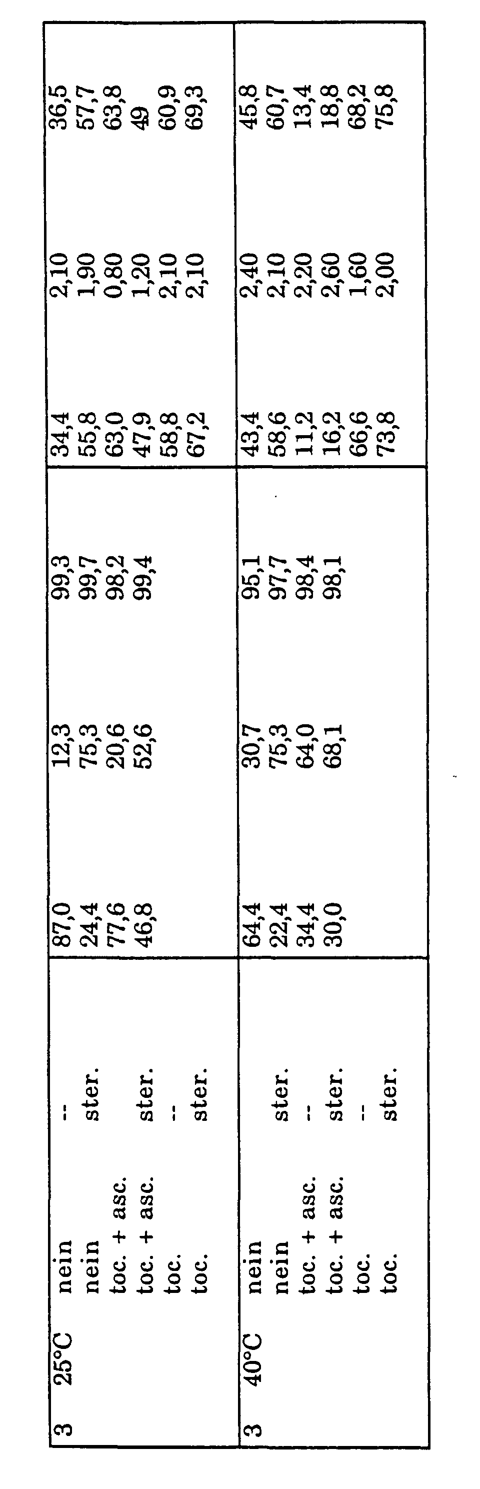

## Patentansprüche

1. Mischmicellen, die aus
a) einem Phosphatid mit gesättigten, verzweigten Fettsäureresten und
b) einem Gallensäuresalz oder Dihexanoyllecithin gebildet sind.

2. Mischmicellen gemäss Anspruch 1, in denen die verzweigten Fettsäurereste 14 bis 20 C-Atome in gerader Kette enthalten.

3. Mischmicellen gemäss Anspruch 1, in denen die verzweigten Fettsäurereste eine ungerade Anzahl von C-Atomen in gerader Kette aufweisen.

4. Mischmicellen gemäss den Ansprüchen 1-3, in denen die verzweigten Fettsäurereste durch eine Methyl- oder Aethylgruppe einfach substituierte Fettsäurereste sind.

5. Mischmicellen gemäss den Ansprüchen 1-4, deren Phasenübergangstemperatur unterhalb 20°C liegt.

6. Mischmicellen gemäss den Ansprüchen 1-5, in denen das Phosphatid
1,2-Di(10-methylnonadecanoyl)-sn-glycero-3-phosphocholin,
1,2-Di(10-methylstearoyl)-sn-glycero-3-phosphocholin,
1,2-Di(8-methylheptadecanoyl)-sn-glycero-3-phosphocholin,
1,2-Di(8-methylpalmitoyl)-sn-glycero-3-phosphocholin, oder
1,2-Di(10-ethylstearoyl)-sn-glycero-3-phosphocholin,
oder ein Enantiomer davon, oder ein entsprechendes Phospho-ethanolamin ist.

7. Mischmicellen gemäss den Ansprüchen 1-6, die ein Gallensäuresalz enthalten.

8. Mischmicellen gemäss den Ansprüchen 1-7, die einen pharmazeutischen Wirkstoff oder ein Kosmetikum enthalten.

9. Wässrige Kompositionen, enthaltend Mischmicellen gemäss den Ansprüchen 1-7, und Lyophilisate davon.

10. Verfahren zur Herstellung von Mischmicellen gemäss den Ansprüchen 1-7 durch Vermischen der einzelnen Bestandteile oder dadurch, dass man die Micellbildner und einen gegebenenfalls beizufügenden Wirkstoff in einem organischen Lösungsmittel, z.B. Methylenchlorid, Aethanol und/oder Methanol löst, das Lösungsmittel, zweckmässig in einem Rotationsverdampfer, entfernt und den Rückstand in Wasser oder einer wässrigen Lösung von pharmazeutischen Hilfsstoffen, wie Pufferstoffen oder Isotonisierungsmitteln löst; oder die freien Gallensäuren, gegebenenfalls unter Zusatz pharmazeutischer Hilfsstoffe und/oder wasserlöslicher Wirkstoffe, in Wasser suspendiert, das Phosphatid und gegebenenfalls pharmazeutische Wirkstoffe oder Kosmetika in der Suspension dispergiert und das Produkt mit einer Base neutralisiert.

11. Verwendung von Mischmicellen gemäss den Ansprüchen 1-7 zur wässrigen Solubilisierung von in Wasser schlecht oder nicht löslichen pharmazeutischen Wirkstoffen in parenteral verabreichbaren Formulierungen, zur Verbesserung der parenteralen Verträglichkeit bei wasserlöslichen Wirkstoffen oder zur Verbesserung der Hautpenetration von pharmazeutischen Wirkstoffe oder Kosmetika.

## Claims

1. Mixed micelles which are formed from
a) a phosphatide with saturated, branched fatty acid residues and
b) a cholanic acid salt or dihexanoyllecithin.

2. Mixed micelles according to claim 1, in which the branched fatty acid residues contain 14 to 20 C atoms in a straight chain.

3. Mixed micelles according to claim 1, in which the branched fatty acid residues have an odd number of C atoms in a straight chain.

4. Mixed micelles according to claims 1-3, in which the branched fatty acid residues are fatty acid residues singly substituted by a methyl or ethyl group.

5. Mixed micelles according to claims 1-4, the phase transition temperature of which lies below 20°C.

6. Mixed micelles according to claims 1-5, in which the phosphatide is
1,2-di(10-methylnonadecanoyl)-sn-glycero-3-phosphocholine,
1,2-di(10-methylstearoyl)-sn-glycero-3-phosphocholine,
1,2-di(8-methylheptadecanoyl) -sn-glycero-3-phosphocholine,
1,2-di(8-methylpalmitoyl) -sn-glycero-3-phosphocholine, or
1,2-di(10-ethylstearoyl)-sn-glycero-3-phosphocholine
or an enantiomer thereof or a corresponding phospho-ethanolamine.

7. Mixed micelles according to claims 1-6, which contain a cholanic acid salt.

8. Mixed micelles according to claims 1-7, which contain a pharmaceutically active substance or a cosmetic.

9. Aqueous compositions, containing mixed micelles according to claims 1-7, and lyophilizates thereof.

10. A process for the manufacture of mixed micelles according to claims 1-7, by mixing the individual components or dissolving the micelle formers and an optionally added active substance in an organic solvent, e.g. methylene chloride, ethanol and/or methanol, removing the solvent, conveniently on a rotary evaporator, and dissolving the residue in water or an aqueous solution of pharmaceutical adjuvants, such as buffers or isotonizing agents; or suspending the free cholanic acids in water, optionally with the addition of pharmaceutical adjuvants and/or water-soluble active substances, dispersing the phosphatide and optionally pharmaceutically active substances or cosmetics in the suspension and neutralizing the product with a base.

11. The use of mixed micelles according to claims 1-7 for the aqueous solubilization of pharmaceutically active substances which are difficultly soluble or insoluble in water in parenterally administerable formulations, for improving the parenteral compatibility in the case of water-soluble active substances or for improving the skin penetration of pharmaceutically active substances or cosmetics.

## Revendications

1. Micelles mixtes, qui sont formées à partir
a) d'un phosphatide à restes d'acides gras saturés, ramifiés et
b) de dihexanoyllécithine ou d'un sel d'acide biliaire.

2. Micelles mixtes selon la revendication 1, dans lesquelles les restes d'acides gras ramifiés contiennent de 14 à 20 atomes de carbone dans la chaîne droite.

3. Micelles mixtes selon la revendication 1, dans lesquelles les restes d'acides gras ramifiés comportent un nombre impair d'atomes de carbone dans la chaîne droite.

4. Micelles mixtes selon les revendications 1-3, dans lesquelles les restes d'acides gras ramifiés sont monosubstitués par un groupe méthyle ou éthyle.

5. Micelles mixtes selon les revendications 1-4, dont la température de transition de phase est inférieure à 20°C.

6. Micelles mixtes selon les revendications 1-5, dans lesquelles le phosphatide est
la 1,2-di(10-méthylnonadécanoyl)-sn-glycéro-3-phosphocholine,
la 1,2-di(10-méthylstéaroyl)-sn-glycéro-3-phosphocholine,
la 1,2-di(8-méthylheptadécanoyl)-sn-glycéro-3-phosphocholine,
la 1,2-di(8-méthylpalmitoyl)-sn-glycéro-3-phosphocholine ou
la 1,2-di(10-éthylstéaroyl)-sn-glycéro-3-phosphocholine,
ou un énantiomère d'un tel composé, ou une phospho-éthanolamine correspondante.

7. Micelles mixtes selon les revendications 1-6, qui contiennent un sel d'acide biliaire.

8. Micelles mixtes selon les revendications 1-7, qui contiennent une substance active pharmaceutique ou un cosmétique.

9. Compositions aqueuses contenant des micelles mixtes selon les revendications 1-7, et lyophilisats de celles-ci.

10. Procédé pour la préparation de micelles mixtes selon les revendications 1-7, par mélange des composants individuels ou par le fait qu'on dissout les générateurs de micelles et une substance active éventuellement à ajouter, dans un solvant organique, par exemple, le chlorure de méthylène, l'éthanol et/ou le méthanol, on élimine le solvant, convenablement dans un évaporateur rotatif et on dissout le résidu dans de l'eau ou une solution aqueuse d'adjuvants pharmaceutiques, tels que des substances tampons ou des agents d'isotonie ; ou bien on met en suspension dans de l'eau les acides biliaires libres, éventuellement avec addition d'adjuvants pharmaceutiques et/ou de substances actives hydrosolubles, on disperse dans la suspension le phosphatide et éventuellement des substances actives pharmaceutiques ou des cosmétiques, et on neutralise le produit par une base.

11. Utilisation de micelles mixtes selon les revendications 1-7, pour la solubilisation dans l'eau de substances actives pharmaceutiques insolubles ou faiblement solubles dans l'eau, dans des compositions pouvant être administrées par voie parentérale, pour l'amélioration de la tolérance parentérale dans le cas de substances actives hydrosolubles ou pour l'amélioration de la pénétration de substances actives pharmaceutiques ou de cosmétiques dans la peau.
